(19) **Europäisches Patentamt**

**European Patent Office**

**Office européen des brevets**

(11) **EP 1 356 805 B1**

(12) **EUROPÄISCHE PATENTSCHRIFT**

(45) Veröffentlichungstag und Bekanntmachung des Hinweises auf die Patenterteilung:
**14.06.2006 Patentblatt 2006/24**

(51) Int Cl.:
*A61K 8/27* *(2006.01)*        *A61K 8/29* *(2006.01)*
*A61K 8/58* *(2006.01)*        *A61K 8/60* *(2006.01)*
*A61K 8/89* *(2006.01)*        *A61Q 17/04* *(2006.01)*

(21) Anmeldenummer: **03008754.8**

(22) Anmeldetag: **17.04.2003**

(54) **O/W-Emulsion enthaltend eine glycosilierte Organosiliciumverbindung und ein Metalloxid**

O/w emulsion comprising a glycosilated organosilicon and a metal oxide

Emulsion h/e compenant un polysiloxan à groupements glycosides et un oxide metallique

(84) Benannte Vertragsstaaten:
**BE DE FR GB NL**

(30) Priorität: **26.04.2002 DE 10218730**

(43) Veröffentlichungstag der Anmeldung:
**29.10.2003 Patentblatt 2003/44**

(73) Patentinhaber: **Wacker Chemie AG**
**81737 München (DE)**

(72) Erfinder:
 • **Koini, Thomas, Dr.**
  **48176 Saline,**
  **Michigan (US)**
 • **Dahms, Gerd H.**
  **47138 Duisburg (DE)**

(74) Vertreter: **Gössmann, Christoph Tassilo et al**
**Wacker-Chemie GmbH,**
**Zentralbereich PML,**
**Hanns-Seidel-Platz 4**
**81737 München (DE)**

(56) Entgegenhaltungen:
  EP-A- 0 832 644        EP-A- 1 004 614
  EP-A- 1 016 400        FR-A- 2 807 318

 • **PATENT ABSTRACTS OF JAPAN vol. 1995, no. 05, 30. Juni 1995 (1995-06-30) & JP 07 041415 A (SHISEIDO CO LTD), 10. Februar 1995 (1995-02-10)**

**Beschreibung**

Die Erfindung betrifft O/W-Emulsionen und darauf basierende kosmetische Zubereitungen.

Die menschliche Haut lässt sich nur langsam durch vorsichtige Steigerung der Sonnenbestrahlung bis zu einem gewissen Grad an die kurzwellige UV-Strahlung gewöhnen. Die schädigende Wirkung hoher Dosen UV-Lichtes speziell im Bereich zwischen 290 nm und 320 nm Wellenlänge ist allgemein bekannt. Strahlen im Bereich dieses als UVB-Bereich definierten Spektrums verursachen bei längerer Lichtexposition, je nach Hauttyp, ein Erythem, einen Sonnenbrand oder starke Verbrennungen.

Aber auch Strahlen des UVA-Spektrums, das zwischen 320 nm und 400 nm definiert ist, können zu Schädigungen der Bindegewebsfasern führen, wodurch die Haut vorzeitig altert.

Zum Schutz gegen UVA- und UVB-Strahlung sind zahlreiche organische Verbindungen bekannt, die in der Lage sind, die schädliche UV-Strahlung vor dem Auftreten auf der Haut zu neutralisieren bzw. abzuschwächen. Große Nachteile bei der Verwendung dieser Verbindungen bestehen darin, dass sie nur einen kleinen Teil des Lichtspektrums absorbieren, eine ungenügende chemische Stabilität unter Lichteinwirkung zeigen, dass sie in die Haut penetrieren können, dadurch allergische und/oder hormonelle Wirkungen möglich sind (M.Schlumpf, Vortrag, Cosmetic Science Conference at IN-Cosmetics 2001, Düsseldorf, April 2001).

Neben den organischen Verbindungen werden auch anorganische Pigmente als UV-Absorber bzw. UV-Reflektoren in der Kosmetik eingesetzt. Die anorganischen Pigmente, bei denen es sich um Oxide des Titans, Zinks, Eisens oder Aluminiums handelt, zeigen sehr gute Lichtschutzwirkung über ein breites UVA/UVB-Spektrum basierend auf Reflexion, Streuung bzw. Absorption der UV-Strahlung. Sie sind überdies weitestgehend chemisch stabil, d.h. ihre Lichtschutzwirkung bleibt ständig erhalten, es findet keine Penetration in die Haut statt und es werden keine allergischen Reaktionen (Derry, McLaan, Freeman; J. Parenteral & Enteral Nutrition, 7(2), S.131, 1982) ausgelöst. Im Gegenteil: ZnO ist beispielsweise in den USA als Kategorie 1 Hautschutzmittel anerkannt; siehe Federal Register, Teil II, S.34641, 1978. Eine günstige Partikelgröße für derartige Pigmente liegt im Bereich von 100 nm. Auf der Haut aufgetragen, sind die Pigmentpartikel in diesem Größenbereich transparent und zeigen günstige Absorptionseigenschaften.

Nachteilig wirkt sich jedoch die Schwierigkeit der Einarbeitung und Stabilisierung solcher Pigmente in kosmetischen Formulierungen aus.

Arbeitet man Lichtschutzpigmente in herkömmliche Emulsionen ein, dann neigen solche Pigmente zu starker Agglomeratbildung. Diese Agglomerate sind meist derart stabil, dass sie sich beim Auftragen nicht in ihre Primärpartikel zerteilen. Als Folge des schlechten Dispersionsgrades zeigt sich eine unerwünschte weiße Filmbildung auf der Haut (Weißeln). Fernerhin nimmt die gewünschte UV-Absorption des Pigmentes ab, wodurch dessen Effizienz als Lichtschutzmittel negativ beeinträchtigt wird. Ein weiterer Nachteil der Agglomeratbildung besteht darin, dass diese sich beim Verteilen auf der Haut sandig anfühlen. (W.Voss, I.Bunge, SPC, Vol.3, S.25, 2001)

Des weiteren kann es beim herkömmlichen Einarbeiten von Pigmenten zu unerwünschten Löslichkeiten der Pigmentoberfläche in der Wasserphase kommen. Speziell bei ZnO liegt eine pH-Instabilität und Oberflächenreaktivität vor; somit können bei Kontakt mit sauren/alkalischen Formulierungsbestandteilen Zn-Ionen gebildet werden bzw. bei Reaktionen mit Fettsäuren Aggregate entstehen. Es kann daher bei Verwendung von ZnO zur Bildung von Zinkaten kommen, wobei sehr oft mehr als 50 % des ZnO Pigmentes in die wässerige Phase überführt werden kann und somit nicht mehr zur UV-Absorption zur Verfügung steht (siehe Ishii Nobuaki et al., GCI, 2, S.32, 2001).

Um diese Agglomerations- und Reaktivitätsprobleme in den Griff zu bekommen, finden durchwegs gecoatete Pigmente Einsatz. Jedoch ist das Coating sehr oft nicht effizient genug, um die oben beschriebenen Prozesse hintanzuhalten.

Will man Mikropigmente mit einem möglichst feinen Dispersionsgrad in O/W-Emulsionen einarbeiten, so sind aufwendige Prozesse notwendig. Zumeist muss das Pigment in einem Teil der in der Emulsion verwendeten Ölphase vordispergiert werden. Diese von der Emulsion getrennt hergestellte Dispersion wird dann nach Herstellung der Grundemulsion nachträglich zugegeben. Bedingt dadurch, dass sich nur ca. 40 % Pigment in eine Ölphase einarbeiten lassen, ist die Gesamtkonzentration an UV-Strahlen absorbierendem Pigment sehr begrenzt.

Bei üblicherweise in Sonnenschutz-Emulsionen eingesetzten Konzentrationen von 20-30 % Ölanteilen, begrenzt sich die Pigmentkonzentration demnach auf maximal 12 %.

Ein weiterer Nachteil des Einsatzes von vordispergierten Pigmenten ist, dass zur Erzielung eines ausreichenden Dispersionsgrades hydrophobe Dispergatoren in ausreichender Konzentration zugesetzt werden müssen. Überschreitet der für die Vordispersion verwendete hydrophobe Emulgator in der zu formulierenden O/W-Sonnenschutzemulsion eine kritische Konzentration, so verschiebt sich die hydrophile-lipophile Balance (HLB-Wert) in Bereiche, die dann zu einer unerwünschten Inversion der O/W-Emulsionen in eine W/O-Emulsion führt.

Aufgabe der vorliegenden Erfindung ist es, die Nachteile des Standes der Technik zu überwinden und Additive zu finden, welche die direkte Einarbeitung hoher Pigmentkonzentrationen mit konventionell einfachen Herstellungsprozessen erlauben, wobei ein ausreichend hoher Dispersionsgrad des Pigmentes erreicht wird und eine hohe Stabilität der Sonnenschutzemulsion gewährleistet ist, insbesondere die Nachteile des Standes der Technik zu überwinden, um die einfache Formulierung von hocheffizienten und kosmetisch eleganten Lichtschutzemulsionen auf Pigmentbasis zu ermöglichen.

Die Aufgabe wird durch die Erfindung gelöst.

Gegenstand der Erfindung ist eine O/W-Emulsion, die zumindest eine Glycosidrest aufweisende Organosiliciumverbindung und zumindest ein Metalloxid enthält gemäß Anspruch 1.

Bei den Metalloxiden handelt es sich vorzugsweise um die Oxide des Titans, Zinks, Eisens oder Aluminiums, bevorzugt Zink- und Titanoxide. Sie werden in Mengen von vorzugsweise 0,1 - 50 Gew.% bezogen auf die Zusammensetzung, bevorzugt 1 - 30 Gew.%, besonders bevorzugt 2 - 20 Gew.%, eingesetzt. Die Metalloxide weisen eine durchschnittliche Partikelgröße von vorzugsweise 5 - 1000 nm, bevorzugt 5 - 400 nm, besonders bevorzugt 5 - 100 nm, auf.

Die Glycosidrest aufweisenden Organosiliciumverbindungen sind Glycosidreste aufweisende Organosiliciumverbindungen aus Einheiten der Formel

$$R_a R^1{}_b SiO_{\frac{4-a-b}{2}} \qquad (I),$$

R gleich oder verschieden sein kann und Wasserstoffatom oder organischer Rest bedeutet,

a 0, 1, 2 oder 3 ist,

b 0, 1, 2 oder 3 ist und

$R^1$ gleich oder verschieden sein kann und einen Rest der Formel

$$Z\text{-}(R^2O)_C\text{-}R^3\text{-} \qquad (II)$$

bedeutet, worin

Z einen Glycosidrest bedeutet, der aus 1 bis 10, vorzugsweise 1 bis 4, besonders bevorzugt 1 bis 2, Monosaccharideinheiten aufgebaut ist,

$R^2$ gleich oder verschieden sein kann und Alkylenrest bedeutet,

c 0 oder eine Zahl von 1 bis 20, bevorzugt 0 oder eine Zahl von 1 bis 15, besonders bevorzugt 0 oder eine Zahl von 1 bis 4, ist und

$R^3$ Alkylenrest bedeutet,

mit der Maßgabe, dass die Summe aus a und b kleiner oder gleich 3-ist und Organosiliciumverbindung aus Einheiten der Formel (I) pro Molekül mindestens einen Rest $R^1$ enthält.

Bevorzugt handelt es sich bei Rest R um gegebenenfalls substituierte Kohlenwasserstoffreste mit 1 bis 18 Kohlenstoffatomen, wobei Alkylreste mit 1 bis 4 Kohlenstoffatomen, insbesondere der Methylrest, besonders bevorzugt sind.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, Octadecylreste, wie der n-Octadecylrest; Alkenylreste, wie der Vinyl-, Allyl, n-5-Hexenyl-, 4-Vinylcyclohexyl- und der 3-Norbornenylrest; Cycloalkylreste, wie Cyclopentyl-, Cyclohexyl-, 4-Ethylcyclohexyl-, Cycloheptylreste, Norbornylreste und Methylcyclohexylreste; Arylreste, wie der Phenyl-, Biphenylyl-, Naphthyl- und Anthryl- und Phenanthrylrest; Alkarylreste, wie o-, m-, p-Tolylreste, Xylylreste und Ethylphenylreste; Aralkylreste, wie der Benzylrest, der α- und der β-Phenylethylrest.

Beispiele für Monosaccharide, aus denen die Glycosidreste Z aufgebaut sein können, sind Hexosen und Pentosen, wie Glucose, Fructose, Galactose, Mannose, Talose, Allose, Altrose, Idose, Arabinose, Xylose, Lyxose und Ribose, wobei Glucose besonders bevorzugt ist.

Beispiele für Alkylenreste sind Methylen-, Ethylen-, Propylen-, Butylen-, Pentylen-, Hexylen-, Heptylen-, Octylen-, Nonylen-, Decylen- und Octadecylenreste.

Bevorzugt handelt es sich bei Rest $R^2$ um den Ethylenrest und den 1,2-Propylenrest, wobei der Ethylenrest besonders bevorzugt ist.

Bevorzugt handelt es sich bei Rest $R^3$ um lineare Alkylenreste mit 2 bis 20 Kohlenstoffatomen, besonders bevorzugt um lineare Alkylenreste mit 2 bis 8 Kohlenstoffatomen, insbesondere um den n-Propylenrest.

Beispiele für Reste $R^1$ sind

$$G\text{-}CH_2CH_2CH2\text{-},$$

G-(CH$_2$CH$_2$O)-CH$_2$CH$_2$CH$_2$-,

G-(CH$_2$CH$_2$O)$_2$-CH$_2$CH$_2$CH$_2$-,

G-(CH$_2$(CH$_3$-)CHO)-CH$_2$CH$_2$CH$_2$-,

G-(CH$_2$(CH$_3$-)CHO)$_2$-CH$_2$CH$_2$CH$_2$-,

G-(CH$_2$CH$_2$O)-CH$_2$CH$_2$(CH$_3$-)CHCH$_2$-,

G-(CH$_2$CH$_2$O)$_2$-CH$_2$CH$_2$(CH$_3$-)CHCH$_2$-,

wobei G einen Glycosidrest (C$_6$H$_{11}$O$_6$-) bedeutet,

G$_2$-CH$_2$CH$_2$CH$_2$-,

G$_2$-(CH$_2$CH$_2$O)-CH$_2$CH$_2$CH$_2$-,

G$_2$-(CH$_2$CH$_2$O)$_2$-CH$_2$CH$_2$CH$_2$-

G$_2$-(CH$_2$(CH$_3$-)CHO)-CH$_2$CH$_2$CH$_2$-,

G$_2$-(CH$_2$(CH$_3$-)CHO)$_2$-CH$_2$CH$_2$CH$_2$-,

G$_2$-(CH$_2$CH$_2$O)-CH$_2$CH$_2$(CH$_3$-)CHCH$_2$-

und

G$_2$-(CH$_2$CH$_2$O)$_2$-CH$_2$CH$_2$(CH$_3$-)CHCH$_2$-,

wobei G$_2$ einen aus zwei Glucoseeinheiten aufgebauten Glycosidrest bedeutet.

Bevorzugt handelt es sich bei Rest R$^1$ um G-CH$_2$CH$_2$CH$_2$-, G-(CH$_2$CH$_2$O)-CH$_2$CH$_2$CH$_2$-, G$_2$-CH$_2$CH$_2$CH$_2$- und G$_2$-(CH$_2$CH$_2$O)- CH$_2$CH$_2$CH$_2$-, wobei G-(CH$_2$CH$_2$O)-CH$_2$CH$_2$CH$_2$-, und G$_2$-(CH$_2$CH$_2$O)- CH$_2$CH$_2$CH$_2$- besonders bevorzugt sind und G einen Glucosidrest (C$_6$H$_{11}$O$_6$-) und G$_2$ einen aus zwei Glucoseeinheiten aufgebauten Glycosidrest bedeutet.

Bevorzugt handelt es sich bei den erfindungsgemäßen, Glycosidresten aufweisenden Organosiliciumverbindungen um solche der Formel

$$R^1_x R_{3-x} SiO-[(SiRR^1O)_m-(SiR_2O)_n]_y-SiR_{3-x}R^1_x \qquad (III),$$

worin R und R$^1$ die obengenannte Bedeutung haben,

m gleich oder verschieden sein kann und 0 oder eine Zahl von 1 bis 200, bevorzugt 0 oder eine Zahl von 1 bis 100, besonders bevorzugt 0 oder eine Zahl von 1 bis 50, ist,

n gleich oder verschieden sein kann und 0 oder eine Zahl von 1 bis 1000, bevorzugt 0 oder eine Zahl von 1 bis 500, besonders bevorzugt 0 oder eine Zahl von 1 bis 100, ist,

x 0 oder 1 ist und

y 0 oder eine Zahl von 1 bis 1200, bevorzugt 0 oder eine Zahl von 1 bis 600, besonders bevorzugt 0 oder eine Zahl von 1 bis 100, ist,

mit der Maßgabe, daß die Verbindung der Formel (III) mindestens einen Rest R$^1$ aufweist.

Ist in den Glycosidresten aufweisenden Organosiliciumverbindungen gemäß Formel (III) m durchschnittlich verschieden 0, bedeutet x bevorzugt 0.

Ist in den Glycosidresten aufweisenden Organosiliciumverbindungen gemäß Formel (III) x durchschnittlich verschieden 0, ist m bevorzugt 0.

Obwohl durch Formel (III) nicht dargestellt, können bis zu 10 Molprozent der Diorganosiloxaneinheiten durch andere Siloxaneinheiten, wie beispielsweise RSiO$_{3/2}$-, R$^1$SiO$_{3/2}$- und/oder SiO$_{4/2}$-Einheiten, ersetzt sein, wobei R und R$^1$ die oben dafür angegebene Bedeutung haben.

Die erfindungsgemäßen, Glycosidreste aufweisenden Organosiliciumverbindungen können nach verschiedenen Verfahren hergestellt werden, die in DE 43 06 041 A1 oder US 5,831,080 beschrieben sind.

Weitere geeignete Glycosidreste aufweisende Organosiliciumverbindungen sind in EP 1004614A1 (Wacker-Chemie GmbH), EP 0879840, JP5186596 beschrieben.

Die erfindungsgemäßen Glycosidreste aufweisende Organosiliciumverbindungen werden vorzugsweise in Mengen von 0,1 und 70 Gew.-% bezogen auf die eingesetzte Metalloxidkonzentration, bevorzugt zwischen 0,2 und 50 Gew.-%, besonders bevorzugt zwischen 1 und 20 Gew.-%, je nach eingesetzter Metalloxidkonzentration eingesetzt.

Die erfindungsgemäßen Glycosidreste aufweisenden Organosiliciumverbindungen werden vorzugsweise in einem Öl gelöst.

Überraschend ist auch die Tatsache, dass unabhängig von dem ionischen Charakter der zur Stabilisierung der O/W-Emulsion eingesetzten Emulgatoren gleich gute Ergebnisse erzielt werden. Auch eine Variation der Ölpolarität der in der O/W-Emulsion eingesetzten Öle, führt zu keinerlei Einbußen der Effizienz der eingesetzten Glycosidreste aufweisenden Organosiliciumverbindungen. Als Öle kommen Siliconöle und deren Derivate (z.B. hoch- und niedrigviskose Organopolysiloxane wie Dimethylpolysiloxane, Methylphenylpolysiloxane, Methylhydrogenpolysiloxane und Dimethylsiloxan-methylphenylsiloxan-Copolymer, Cyclische Siloxane wie Octamethylcyclotetrasiloxan, Decamethylcyclopentasiloxan, Dodecamethylcyclohexasiloxan, Tetramethyltetrahydrogencyclotetrasiloxane, Silicon-Kautschuke wie Dimethylpolysiloxane mit einem hohen Polymerisierungsgrad und Dimethylsiloxan-methylphenyl siloxan-Copolymere mit hohem Polymerisierungsgrad und Cyclosiloxan-Lösungen von Silicon-Rubber, Trimethylsiloxysilicat, Cyclosiloxan-Lösungen von Trimethylsiloxysilicat, höhere alkoxy-modifizierte Silicone wie Stearoxysilicone, höhere Fettsäure-modifizierte Silicone, alkyl-modifizierte Silicone, Amino-modifizierte Silicone, fluoro- modifizierte Silicone, und Siliconharzlösungen; diese Silicone sind nicht strukturell eingeschränkt, sondern können jegliche geradkettige, verzeigte, untereinander vernetzte und cyclische Struktur besitzen),

Öle/Wachse pflanzlichen und tierischen Ursprungs (z.B. Avocadoöl, Leinsamenöl, Mandelöl, Carnaubawachs, Leberöl, Candelilawachs, Tallow-Derivate, Aprikosenöl, Hydrogeniertes Öl, Weizenkeimöl, Sesamöl, Reiskeimöl, Zuckerrohrwachs, Jojobaöl, Soyabohnenöl, Teeöl, Fett, Rapsöl, Palmöl, Rizinusöl, Sonnenblumenöl, Jojobawachs, Kokosnussöl, Fettsäureglyceride, hydrogenierte Öle, Erdnußöl, Lanolin & Derivate, Hexyllaurat), Esteröle (z.B. Diisobutyladipat, 2-Hexyldecyladipat, Di-2-heptylundecyladipate, N-alkylglycolmonoisostearate, Isocetylisostearat, Trimethylolpropan-Triisostearinsäure, Ethylenglycol, Di-2-ethylhexansäureester, Cetyl-2-ethylhexanoat, Trimethylolpropan, Tri-2-ethylhexansäureester, Pentaerythritol-tetra-2-ethylhexansäureester, Cetyloctanoat, Octyldodecylgumester, Oleyloleat, Octyldodecyloleat, Decyloleat, Neopentylglycol-dicaprylsäureester, Triethylcitrat, 2-Ethylhexylcinnamat, Amylacetate, Ethylacetat, Butylacetat, Isocetylstearat, Butylstearat, Diisopropylsebacat, Di-2-ethylhexylsebacat, Cetyllactat, Myristyllactat, Isopropylpalmitat, 2-Ethylhexylpalmitat, 2-Hexyldecylpalmitat, 2-Heptylundecylpalmitat, Cholesteryl-12-hydroxystearat, Dipentaerythritolfettsäureester, Isopropylmyristat, Octyldodecylmyristat, 2-Hexyldecylmyristat, Myristylmyristat, Hexyldecyldimethyloctanoat, Ethyllaurat, Hexyllaurat, N-Läuroyl-L-glutaminsäure, 2-Octyldodecylester und Diisostearylmaleinsäure), flüssige Fettalkohole (z.B. Laurylalkohol, Myristylalkohol, Palmitylalkohol, Stearylalkohol, Behenylalkohol, Hexadecylalkohol, Oleylalkohol, Isostearylalkohol, Octadodecanol, Cetylstearylalkohol, 2-Decyltetradecinol, Cholesterol, Phytosterol, Monostearylglycerinether (Batylalkohol), Monooleylglycerylether (Cerylalkohol)), paraffinbasische Öle/Wachse (z.B. Ozokerite, Squalan, Ceresin, Paraffin, Paraffin-Wachs, Flüssiges Paraffin, Pristan, Polyisobutylen, microkristallines Wachs and Vaseline), höhere Fettsäuren (z.B. Laurinsäure, Stearinsäure, Behreninsäure, Undecensäure, Oleinsäure, Linolsäure, Eicosapentaenoische Säure (EPA), Docosahexaenoische Säure (DHA), Isostearinsäure)in Frage. Als Öle sind bevorzugt Cyclomethicone, Hexamethyldisiloxan und Dimethicone, besonders bevorzugt Cyclohexasiloxan, Cyclopentasiloxan, Cyclotetrasiloxan und niedrigviskose Dimethicone.

Diese Öle können in beliebigen Mischungen miteinander eingesetzt werden.

Die Öle werden vorzugsweise im Verhältnis 9 Gewichtsteile Öl auf 1 Gewichtsteil Glycosidreste aufweisende Organosiliciumverbindungen, bevorzugt im Verhältnis 7 Gewichtsteile Öl auf 1 Gewichtsteil Glycosidreste aufweisende Organosiliciumverbindungen, im Verhältnis 2 Gewichtsteile Öl auf 1 Gewichtsteil Glycosidreste aufweisende Organosiliciumverbindungen, besonders bevorzugt im Verhältnis 4 Gewichtsteile Öl auf 1 Gewichtsteil Glycosidreste aufweisende Organosiliciumverbindungen eingesetzt.

Die Qualität des in der Emulsion erzielten Dispersionsgrades wird auch durch organische UV-Filter wie z.B. vom Benzoesäure-Typ, Anthranylsäure-Typ, Salicylsäure-Typ, Succinsäure-Typ, Benzophenon-Typ, Uranylsäure-Typ, Dibenzoylsäure-Typ, Cinnamylsäure-Typ nicht negativ beeinflusst.

Auch Modifikationen der Wasserphase beeinträchtigen nicht die Effizienz der O/W-Emulsion, die Glycosidreste aufweisende Organosiliciumverbindungen (in Lösungsmittel vorgelöst) zusammen mit Mikropigmenten enthält.

Zur Herstellung einer Formulierung können ebenso die Inhaltsstoffe verwendet werden, welche normalerweise bei kosmetischen Zubereitungen Verwendung finden, wie Wasser, Filmbildner, öllösliche Verbindungen, Harze, UV-Absorber, Feuchtigkeitsspender, antiseptische Mittel, Konservierer, Parfüm, Salze, Antioxidantien, pH-Wert-Regulierer, Komplexbildner, Entzündungshemmer, Hautverschönerungsmittel (z.B. Hautbleicher, Zellaktivatoren, Verbesserer für raue und trockene Haut, Anreger der Blutzirkulation), Vitamine, Aminosäuren, Nukleinsäuren, Hormone, solange sie keine störenden Einflüsse auf die erwünschten Effekte zeigen.

Emulgatoren können anionisch, kationisch, nicht-ionisch und amphoter in ihrer Natur sein. Beispiele verwendbarer anionischer Emulgatoren sind verseifte Fettsäuren, z.B. Natriumstearat / Triethanolamin-palmitat, Alkylethercarbonsäuren und ihre Salze, Salze von Aminosäure-Fettsäure-Kondensaten, Alkansulfonate, Alkensulfonate, sulfonierte Fettsäureester, Alkylsulfate, Sulfate höherer sec. Alkohole, Alkyl- und Arylethersulfate, Fettsäureethersulfate, Fettsäurealkylamidsulfate, Ethersulfate, Alkylphosphate, Etherphosphate, Alkylaryletherphosphate, Amidphosphate, aktive Vertreter vom N-Acylaminosäuretyp.

Beispiele verwendbarer kationischer Emulgatoren sind Aminsalze, z.B. Alkylaminsalze, Polyamine und nichtalkoholische aminische Fettsäurederivate, quarternäre Alkylammoniumsalze, quarternäre Arylammoniumsalze. Beispiele verwendbarer nichtionischer Emulgatoren sind Sörbitanfettsäureester, Glycerinfettsäureester, Polyglycerinfettsäureester, Propylenglycolfettsäureester, Polyethylenglycolfettsäureester, Polyoxyethylenalkylether, Polyoxypropylenalkylether, Polyoxyethylenalkylphenylether, Polyoxyethylenfettsäureester, Polyoxyethylensorbitanfettsäurester, Polyoxyethylensorbitolfettsäurester, Polyoxyethyleneglycerinfettsäurester, Polyoxyethylenpropylenglycolfettsäurester, Polyoxyethylene von Rizinusoel und hydrogeniertem Rizinusoel, Polyoxyethylenephytostanolether, Polyoxyethylenephytosterolether, Polyoxyethylenecholestanolether, Polyoxyethylenecholesterolether, Polyoxyalkylen-modifizierte Organopolysiloxanes, Organopolysiloxane modifiziert mit Polyoxyalkyl- and Alkylgruppen, Alkanolamide, Zuckerether und Zuckeramide, Fettalkohole.

Beispiele verwendbarer amphoterer Emulgatoren sind Betainaminocarboxylate und Imidazolderivate.

Als Emulgatoren werden vorzugsweise Sorbitanfettsäureester, Glycerinfettsäureester, Polyglycerinfettsäureester, Propylenglycolfettsäureester, Polyethylenglycolfettsäureester, Polyoxyethylenalkylether, Polyoxypropylenalkylether, Polyoxyethylensorbitanfettsäurester, Polyoxyethylensorbitolfettsäurester, Polyoxyethyleneglycerinfettsäurester, Polyoxyethylenpropylenglycolfettsäurester, Polyoxyethylene von hydrogeniertem Rizinusoel, Polyoxyalkylen-modifizierte Organopolysiloxanes, Organopolysiloxane modifiziert mit Polyoxyalkyl- und Alkylgruppen, Fettalkohole, besonders bevorzugt Alkylethercarbonsäuren und ihre Salze, Alkylsulfate, Glycerinfettsäureester, Polyethylenglycolfettsäureester, Sucrosefettsäureester, Polyoxyalkylen-modifizierte Organopolysiloxanes, Organopolysiloxane modifiziert mit Polyoxyalkyl- und Alkylgruppen, Fettalkohole.

Ein weiterer Gegenstand der Erfindung ist ein Verfahren zur Herstellung der O/W-Emulsion, wobei eine O/W-Emulsion hergestellt wird, zu der bei einer Temperatur von unter 50 °C die in einem Lösungsmittel gelöste Glycosidrest aufweisende Organosiliciumverbindung mit dem Metalloxid hinzugefügt wird.

Beim Verfahren zur Herstellung der erfindungsgemäßen O/W-Emulsionen, wird das zu verwendende Mikropigment zusammen mit der Glycosidrest aufweisenden Organosiliciumverbindung (in Lösungsmittel gelöst) nach Abkühlen der Emulsion bei einer Temperatur unter 50 ° C zugegeben. Besonders bevorzugt ist bei diesem Verfahren die Zugabe im Bereich von 20 bis 45° C.

Ein weiterer Gegenstand der Erfindung ist eine kosmetische Zubereitung auf der Basis der erfindungsgemäßen O/W-Emulsion.

Die kosmetische Zubereitung wird bevorzugt als Lotion oder Creme zubereitet.

Diese kosmetische Zubereitung ist besonders als Sonnenschutzmittel geeignet.

Ein weiterer Gegenstand ist die Verwendung der kosmetischen Zubereitung zum Sonnenschutz.

Beispiele:

Formulierungen mit ZnO:

So ermöglicht z.B. der Einsatz von nur 1% Glycosidreste aufweisende Organosiliciumverbindung (in Lösungsmittel vorgelöst) das problemlose Einarbeiten von 20 % ZnO in eine konventionell hergestellte O/W-Rahmenformulierung. Verzichtet man auf den Einsatz der Glycosidrest aufweisenden Organosiliciumverbindung (in Lösungsmittel vorgelöst), invertieren die O/W-Rahmenformulierungen bereits bei einer Einsatzkonzentration von 10 % ZnO zu W/O-Emulsionen.

Eine hohe Pigmentkonzentration:

Die Phaseninversion, die durch eine kritische Konzentration von 10 % ZnO hervorgerufen wird, kann wie folgt erklärt werden. Bei der Zugabe von ZnO in die Emulsion tritt dieses Pigment in Wechselwirkung mit dem verwendeten Emulgator. Dabei werden an der stark polaren ZnO Oberfläche die hydrophilen Tenside adsorbiert, die für die Stabilisierung der O/W-Emulsion verantwortlich sind. Die Menge der adsorbierten hydrophilen Tenside hängt dabei von der Konzentration des eingesetzten ZnO-Pigmentes ab. Mit anderen Worten, bei steigender Zinkoxid-Konzentration verarmt die O/W-Emulsion an hydrophilem Tensid. Dadurch nimmt der HLB-Wert der eingesetzten Emulgatormischung ebenfalls ab. Sobald der HLB-Wert unter 6 fällt, tendiert das System zur Inversion in eine W/O-Emulsion. -

Wird jedoch das Zinkoxid-Pulver zusammen mit der Glycosidrest aufweisenden Organosiliciumverbindung (in Lösungsmittel vorgelöst) in die Emulsion eingearbeitet, zieht die Glycosidrest aufweisende Organosiliciumverbindung (in Lösungsmittel vorgelöst) trotz ihres hohen Molekulargewichtes und ihrer relativ geringen Hydrophilie überraschenderweise schneller auf die ZnO-Oberfläche auf, als die niedrig molekularen hydrophilen Emulgatoren, die in O/W-Emulsionssystemen eingesetzt werden. Durch die hohe Affinität der Glycosidrest aufweisenden Organosiliciumverbindung (in Lösungsmittel vorgelöst) zur Pigmentoberfläche wird diese fast vollständig abgeschirmt.

pH-Wert:

Durch diesen guten Abschirmeffekt wird außerdem die unerwünschte Bildung von Zinkat fast vollständig unterdrückt. Der Nachweis der alkalisch reagierenden Zinkate, die sich von der ZnO-Oberfläche bilden, kann über die pH-Wert-Messung erfolgen. Vergleicht man wie in Tabelle 1 dargestellt die pH-Werte der Emulsionen mit und ohne Glycosidrest aufweisende Organosiliciumverbindung (in Lösungsmittel vorgelöst), so sieht man deutlich einen Anstieg des pH-Wertes mit steigender ZnO-Konzentration in der Emulsion ohne Glycosidrest aufweisende Organosiliciumverbindung (in Lösungsmittel vorgelöst), während der Anstieg des pH-Wertes bei der Emulsion mit Glycosidrest aufweisende Organosiliciumverbindung (in Lösungsmittel vorgelöst) kaum merklich zunimmt.

| ZnO Konzentration (Gew. %) | pH-Wert Emulsion mit GaOSV (in Lösungsmittel vorgelöst) | pH-Wert Emulsion ohne GaOSV (in Lösungsmittel vorgelöst) |
|---|---|---|
| 0 | 5,6 | 5,4 |
| 3 | 5,7 | 6,9 |
| 5 | 5,8 | 7,0 |
| 10 | 5,8 | 7,2 |
| 15 | 5,9 | 7,4 |
| 20 | 6,0 | * |
| **\*Emulsion invertiert** **GaOSV = Glycosidrest aufweisende Organosiliciuraverbindung** | | |

Die starke Bildung von Zinkat in der Emulsion ohne Glycosidrest aufweisende Organosiliciumverbindung (in Lösungsmittel vorgelöst), wirkt aufgrund der dabei entstehenden erhöhten pH-Werte autokatalytisch, d.h. mit zunehmender Lagerdauer wird immer mehr Zinkoxid in Zinkat umgewandelt. Auch dieser Vorgang kann über pH-Wert-Messungen verfolgt werden. Beobachtet man den pH-Wert der Emulsion mit 5 % ZnO, die keine Glycosidrest aufweisende Organosiliciumverbindung (in Lösungsmittel vorgelöst) enthält, so stellt man fest, dass der pH-Wert über die Lagerzeit stark zunimmt, während bei der Emulsion mit Glycosidrest aufweisender Organosiliciumverbindung (in Lösungsmittel vorgelöst) keine pH-Wertzunahme zu beobachten ist.

| Zeit (Tage) | pH-Wert Emulsion mit GaOSV (in Lösungsmittel vorgelöst) | pH-Wert Emulsion ohne GaOSV (in Lösungsmittel vorgelöst) |
|---|---|---|
| 0 | 5,8 | 7,0 |
| 1 | 5,8 | 7,9 |
| 3 | 5,9 | 8,3 |
| 7 | 5,9 | 8,4 |
| 14 | 5,9 | 8,6 |
| **GaOSV = Glycosidrest aufweisende Organosiliciumverbindung** | | |

Die pH-Wert-Analyse zeigt deutlich, wie die ZnO-Pigment-Konzentration bei der Emulsion ohne Glycosidrest aufweisender Organosiliciumverbindung (in Lösungsmittel vorgelöst) über die Zeit abnimmt. Das hat in einer Lichtschutzemulsion zur Folge, dass auch die Lichtschutzwirkung einer solchen Emulsion mit zunehmender Lagerdauer ebenfalls abnimmt. Neben der unerwünschten Abnahme der Pigment-Konzentration kann der hohe pH-Wert auch zu Hautirritationen führen.

Dispergierung, Weißeln:

Der erfindüngsgemäße Zusatz von Glycosidrest aufweisender Organosiliciumverbindung (in Lösungsmittel vorgelöst) führt auch zu einem erheblich besseren Dispersionsgrad des Mikropigmentes ZnO, was bekanntermaßen eine signifikante Steigerung der Lichtschutzwirkung um bis zu 50 % bewirken kann (DE 195 48 015 A1). Neben der Steigerung der Lichtschutzwirkung führt der Einsatz von Glycosidrest aufweisender Organosiliciumverbindung (in Lösungsmittel vorgelöst) zu einer signifikanten Verbesserung der kosmetischen Eigenschaften von Emulsionen auf Basis von Mikropigmenten. So zeigt eine mit 20 Gew.% ZnO beladene Emulsion, die 1,5 Gew. % Glycosidrest aufweisende Organosiliciumverbindung (in Lösungsmittel vorgelöst) enthält, auch auf mediterraner Haut keinerlei weiße Filmbildung.

Formulierungen mit $TiO_2$:

Dispergierung, Weißeln:

Wechselt man von ZnO auf $TiO_2$, vermindert sich keine der positiven Eigenschaften der Glycosidrest aufweisenden Organosiliciumverbindung (in Lösungsmittel vorgelöst). Aufgrund der geringeren Transparenz des $TiO_2$-Mikropigmentes liegt die maximale Konzentration des $TiO_2$-Pigmentes, die noch auf mediterraner Haut nach dem Verteilen zu keiner sichtbaren weißen Filmbildung führt, niedriger. Überraschend ist aber auch hier die sehr hohe Konzentration von 12,5 Gew.% $TiO_2$, die noch transparente Filme bildet. Die überraschend hohe Transparenz der auf der Haut gebildeten Mikropigmentfilme ist zum einen auf den hohen Dispersionsgrad der Mikropigmente und zum anderen auf die Stabilität der Mikropigmentdispersion, während des Verreibens der Lichtschutzemulsion auf der Haut, zurückzuführen. Der überraschend hohe Dispersionsgrad des $TiO_2$ Pigmentes kann sowohl über eine mikroskopische Analyse als auch über die Bestimmung der Partikelgröße mittels Laser-Light-Scattering demonstriert werden. Bei Anwendung der Laser-Light-Scattering-Methode wird für die Mikropigmentverteilung in einer gewöhnlichen Öl-in-Wasser-Emulsion schon bei niedrigem bis mittleren Energieeintrag ein mittlerer Partikeldurchmesser von ~ 200 nm erzielt, verglichen mit professionell hergestellten handelsüblichen $TiO_2$-Dispersionen, deren mittlerer Durchmesser sich im Bereich zwischen 350 und 400 nm bewegt. Die hohe Affinität der Glycosidrest aufweisenden Organosiliciumverbindung (in Lösungsmittel vorgelöst) zum $TiO_2$ garantiert, dass die Dispersion auch unter den Scherkräften beim Verreiben der Emulsion auf der Haut nicht nachteilig beeinflusst wird. Dadurch bedingt kommt es zu einer homogenen Filmbildung auf der Haut, die eine hohe Lichtschutzeffizienz garantiert. Des weiteren wird durch die Scherstabilität während des Verreibens der Lichtschutzemulsion auf der Haut der sehr gute Dispersionsgrad des $TiO_2$ nicht negativ beeinflusst und somit eine überdurchschnittliche Transparenz erreicht.

Hautgefühl:

Auch bei extrem hoher Konzentration an $TiO_2$ von mehr als 10 Gew. % wird bei einem Einsatz von Glycosidrest aufweisender Organosiliciumverbindung (in Lösungsmittel vorgelöst) das sandige Gefühl beim Auftragen auf der Haut, das oftmals schon bei Einsatzkonzentrationen von 3 Gew. % $TiO_2$ als unangenehm wahrgenommen wird, nicht empfunden.

pH-Wert:

Die hohe Affinität der Glycosidrest aufweisenden Organosiliciumverbindung (in Lösungsmittel vorgelöst) bewirkt auch die Bildung eines kohärenten Schutzfilmes an ungenügend beschichtetem $TiO_2$. Es ist bekannt, dass gerade mikrofeines $TiO_2$ als Halbleiter unter Lichteinwirkung ein starkes Oxidationsmittel gegenüber organischen Substanzen darstellt. Die entstehenden Oxidationsprodukte bewirken einen pH-Wertabfall, der in $TiO_2$-haltigen Emulsionen mit Glycosidrest aufweisender Organosiliciumverbindung (in Lösungsmittel vorgelöst) nicht beobachtet wird.

| Beispiel | 1 | 2 |
|---|---|---|
| Phase A | | |
| Polyglyceryl-3 methyl glucose isostearat | 4,00 | 4,00 |
| Octyl stearat | 8,00 | 8,00 |
| Cetearyl isononanoat | 3,00 | 3,00 |
| Isoamyl p-methoxycinnamat | 9,00 | 9,00 |
| Tocopherol acetat | 1,00 | 1,00 |
| Butyl methoxydibenzoylmethan | 0,50 | 0,50 |
| Phase B | | |
| Wasser | 63,70 | 59,70 |
| Glycerin | 5,00 | 5,00 |

Tabelle fortgesetzt

| Phase B | | |
|---|---|---|
| Xanthangummi | 0,30 | 0,30 |
| **Phase C** | | |
| 20% GaOSV in cyclomethicon | 0,00 | 4,00 |
| Titandioxid | 4,00 | 4,00 |
| **Phase D** | | |
| Konservierungsmittel | q.s. 100,00 | q.s. 100,00 |
| **GaOSV = Glycosidrest aufweisende Organosiliciumverbindung** | | |

| Zeit (Tage) | pH-Wert Emulsion 1 ohne GaOSV (in Lösungsmittel vorgelöst) | pH-Wert Emulsion 2 mit GaOSV (in Lösungsmittel vorgelöst) |
|---|---|---|
| 1 | 7,0 | 7,0 |
| 10 | 6,5 | 6,9 |
| 36 | 5,6 | 6,7 |
| 150 | 5,2 | 6,7 |
| **GaOSV = Glycosidrest aufweisende Organosiliciumverbindung** | | |

Formulierungen:

Formulierungsbestandteile

Die nachfolgenden Beispiele erläutern die Erfindung zusätzlich.

Beispiele

Um die homogene Verteilung des Mikropigmentes zu erreichen, wird zunächst die Grundemulsion bestehend aus der Ölphase A und der Wasserphase B hergestellt. Dazu werden beide Phasen getrennt auf 75°C unter Rühren erhitzt, bis beide Phasen homogen vermischt sind. Danach wird die Ölphase A in die Wasserphase B unter ständigem Rühren einemulgiert und die Emulsion mit einem Rotor-Stator Rührer homogenisiert. Anschließend wird die so erhaltene Emulsion unter Rühren auf 40° C abgekühlt. Der Rührvorgang wird an dieser Stelle unterbrochen. Jetzt wird zunächst das Pigment und anschließend die Glycosidrest aufweisende Organosiliciumverbindung (in Lösungsmittel gelöst) auf die Emulsion gegeben. Danach wird wieder mit einem Rotor-Stator oder schnell drehendem Propeller-Rührer der gesamte Ansatz homogenisiert, bis eine optimale Pigmentverteilung erreicht ist. Die Pigmentverteilung wird mittels Mikroskopie bei Verwendung von polarisiertem Licht durchgeführt. Nachdem eine optimale Pigmentverteilung erreicht ist, wird die Emulsion unter Rühren auf 25 ° C abgekühlt.

Zur Sicherstellung einer optimalen Prozessführung bei industriell hergestellten Emulsionen, wurden die Versuche in einer 2 kg Prozessanlage der Firma IKA LA 2000 V durchgeführt, die in ihrem Aufbau den in der Industrie verwendeten Anlagen entspricht.

**Pigmenthaltige Sonnenschutzemulsionen ohne organische Filter Zinkoxid**

| Beispiel 1 | A |
|---|---|
| Phase A Glycerin stearat, Cetearyl alkohol, Natrium stearoyl lactylat | 5.00 |

Tabelle fortgesetzt

| Beispiel 1 | A |
|---|---|
| Phase A | |
| (BioBase S) | |
| Laureth-23 | 1.00 |
| Cetearyl isononanoat | 8.00 |
| Heptamethylnonan . | 5.00 |
| Tocopherol acetat | 1.00 |
| Phase B | |
| Wasser | 44.20 |
| Glycerin | 5.00 |
| Xanthangummi | 0.30 |
| Phase C | |
| 10% GaOSV in cyclomethicon | 10.00 |
| Zinkoxid | 20.00 |
| Phase D | |
| Konservierungsmittel | q.s. |
| | **100.00** |
| **GaOSV = Glycosidrest aufweisende Organosiliciumverbindung** | |

| Beispiel 2 | B |
|---|---|
| Phase A | |
| Glycerin stearat, Cetearyl alkohol, Natrium stearoyl lactylat (BioBase S) | 5.00 |
| Laureth-23 | 1.00 |
| Tridecyl salicylat | 8.00 |
| Capric/caprylic triglycerid | 5.00 |
| Tocopherol acetat | 1.00 |
| Phase B | |
| Wasser | 44.20 |
| Glycerin | 5.00 |
| Xanthangummi | 0.30 |
| Phase C | |
| 10% GaOSV in Cyclomethicon | 10.00 |
| Zinkoxid | 20.00 |
| Phase D | |
| Konservierungsmittel | q.s. |

Tabelle fortgesetzt

| Phase D | |
|---|---|
| | **100.00** |
| **GaOSV = Glycosidrest aufweisende Organosiliciumverbindung** | |

| **Beispiel 3** | **C** |
|---|---|
| Phase A | |
| Glyceryl stearat, PEG-100 stearat (Arlacel 165) | 1.00 |
| Glyceryl stearat | 2.00 |
| Cetearyl alkohol | 2.00 |
| Laureth-23 | 1.00 |
| Cetearyl isononanoat | 8.00 |
| Heptamethylnonan | 5.00 |
| Tocopherol acetat | 1.00 |
| Phase B | |
| Wasser | 54.20 |
| Glycerin | 5.00 |
| Xanthangummi | 0.30 |
| Phase C | |
| 10% GaOSV in Cyclomethicon | 10.00 |
| Zinkoxid | 10.00 |
| Phase D | |
| Konservierungsmittel | q.s. |
| | **100.00** |
| **Beispiel 4** | **D** |
| Phase A | |
| Glycerin stearat, Cetearyl alkohol, Natrium stearoyl lactylat (BioBase S) | 5.00 |
| Laureth-23 | 1.00 |
| Glyceryl stearat, PEG-100 stearat (Arlacel 165) | 0.00 |
| Glyceryl stearat | 0.00 |
| Cetearyl alkohol | 0.00 |
| Cetearyl isononanoat | 8.00 |
| Heptamethylnonan | 5.00 |
| Tocopherol acetat | 1.00 |
| Phase B | |
| Wasser | 54.20 |
| Glycerin | 5.00 |
| Xanthangummi | 0.30 |

Tabelle fortgesetzt

| Phase C | |
|---|---:|
| 10% GaOSV in Cyclomethicon | 10.00 |
| Zinkoxid | 10.00 |
| Phase D | |
| Konservierungsmittel | q.s. |
| | **100.00** |
| **GaOSV = Glycosidrest aufweisende Organosiliciumverbindung** | |

**Titandioxid**

| Beispiel 5 | E |
|---|---:|
| Phase A | |
| Glycerin stearat, Cetearyl alkohol, Natrium stearoyl lactylat (BioBase S) | 5.00 |
| Laureth-23 | 1.00 |
| Cetearyl isononanoat | 8.00 |
| Heptamethylnonane | 5.00 |
| Tocopherol acetat | 1.00 |
| Phase B | |
| Wasser | 44.20 |
| Glycerin | 5.00 |
| Xanthangummi | 0.30 |
| Phase C | |
| 10% GaOSV in Cyclomethicon | 10.00 |
| Titandioxid | 15.00 |
| Phase D | |
| Konservierungsmittel | q.s. |
| | **100.00** |
| **GaOSV = Glycosidrest aufweisende Organosiliciumverbindung** | |

| Beispiel 6 | F |
|---|---:|
| Phase A | |
| Glycerin stearat, Cetearyl alkohol, Natrium stearoyl lactylat (BioBase S) | 5.00 |
| Läureth-23 | 1.00 |
| Tridecyl salicylat | 8.00 |
| Capric/caprylic triglycerid | 5.00 |
| Tocopherol acetat | 1.00 |
| Phase B | |
| Wasser | 44.20 |

Tabelle fortgesetzt

| Phase B | |
|---|---|
| Glycerin | 5.00 |
| Xanthangummi | 0.30 |
| **Phase C** | |
| 10% GaOSV in Cyclomethicon | 10.00 |
| Titandioxid | 15.00 |
| **Phase D** | |
| Konservierungsmittel | q.s. |
| | **100.00** |
| **GaOSV = Glycosidrest aufweisende Organosiliciumverbindung** | |

| **Beispiel 7** | **G** |
|---|---|
| *Phase A* | |
| Glyceryl stearat, PEG-100 stearat (Arlacel 165) | 1.00 |
| Glyceryl stearat | 2.00 |
| Cetearyl alkohol | 2.00 |
| Laureth-23 | 1.00 |
| Cetearyl isononanoat | 8.00 |
| Heptamethylnonan | 5.00 |
| Tocopherol acetat | 1.00 |
| *Phase B* | |
| Wasser | 44.20 |
| Glycerin | 5.00 |
| Xanthangummi | 0.30 |
| *Phase C* | |
| 10% GaOSV in Cyclomethicon | 10.00 |
| Titandioxid | 15.00 |
| *Phase D* | |
| Konservierungsmittel | q.s. |
| | **100.00** |
| **Beispiel 8** | **H** |
| *Phase A* | |
| Glyceryl stearat, PEG-100 stearat (Arlacel 165) | 1.00 |
| Glyceryl stearat | 2.00 |
| Cetearyl alkohol | 2.00 |
| Laureth-23 | 1.00 |
| Tridecyl salicylat | 8.00 |
| Capric/caprylic triglycerid | 5.00 |
| Tocopherol acetat | 1.00 |

Tabelle fortgesetzt

| Phase B | |
|---|---|
| Wasser | 44.20 |
| Glycerin | 5.00 |
| Xanthangummi | 0.30 |
| Phase C | |
| 10% GaOSV in cyclomethicon | 10.00 |
| Titandioxid | 15.00 |
| Phase D | |
| Konservierungsmittel | q.s. |
| | **100.00** |
| **GaOSV = Glycosidreste aufweisende Organosiliciumverbindung** | |

**Pigmenthaltige Sonneschutzformulierungen mit organischen Filtern**

**Zinkoxid**

| **Beispiel 9** | **A** |
|---|---|
| Phase A | |
| Glycerin stearat, Cetearyl alkohol, Natrium stearoyl lactylat (BioBase S) | 5.00 |
| Laureth-23 | 1.00 |
| Octyl methoxycinnamat | 10.00 |
| Cetearyl isononanoat | 8.00 |
| Heptamethylnonan | 5.00 |
| Tocopherol acetat | 1.00 |
| Phase B | |
| Wasser | 34.20 |
| Glycerin | 5.00 |
| Xanthangummi | 0.30 |
| Phase C | |
| 10% GaOSV in Cyclomethicon | 10.00 |
| Zinkoxid | 20.00 |
| Phase D | |
| Konservierungsmittel | q.s. |
| | **100.00** |
| **GaOSV = Glycosidrest aufweisende Organosiliciumverbindung** | |

**Titandioxid**

| **Beispiel 10** | **F** |
|---|---|
| Phase A | |
| Glycerin stearat, Cetearyl | 5.00 |

Tabelle fortgesetzt

| Beispiel 10 | F |
|---|---|
| Phase A | |
| alkohol, Natrium stearoyl lactylat (BioBase S) | |
| Natrium stearoyl lactylat | 2.50 |
| Tridecyl salicylat | 10.00 |
| Cetearyl isononanoat | 8.00 |
| Heptamethylnonan | 5.00 |
| 4-Methylbenzyliden campher | 3.00 |
| Octocrylen | 3.00 |
| Tocopherol acetat | 1.00 |
| Phase B | |
| Wasser | 41.30 |
| Glycerin | 5.00 |
| Xanthangummi | 0.50 |
| Alkylacrylate/C10-30 alkyl acrylat crosspolymer | 0.20 |
| Phase C | |
| 10% GaOSV in cyclomethicone | 10.00 |
| Titandioxid | 5.00 |
| Phase D | |
| Konservierungsmittel | q.s. |
| | **100.00** |
| **GaOSV = Glycosidreste aufweisende Organosiliciumverbindung** | |

| Beispiel 11 | G |
|---|---|
| Phase A | |
| Glycerin stearat, Cetearyl | 5.00 |
| alkohol, Natrium stearoyl lactylat (BioBase S) | |
| Laureth-23 | 1.00 |
| Octyl methoxycinnamat | 10.00 |
| Cetearyl isononanoat | 8.00 |
| Heptamethylnonan | 5.00 |
| Tocopherol acetat | 1.00 |
| Phase B | |
| Wasser | 39.70 |
| Glycerin | 5.00 |
| Xanthangummi | 0.30 |
| Phase C | |
| 10% GaOSV in Cyclomethicone | 10.00 |
| Titandioxid | 15.00 |
| Phase D | |
| Konservierungsmittel | q.s. |

Tabelle fortgesetzt

| Phase D | |
|---|---|
| | 100.00 |
| **GaOSV = Glycosidreste aufweisende Organosiliciumverbindung** | |

**Patentansprüche**

1. O/W-Sonnenschutz emulsion, **dadurch gekennzeichnet, dass** zumindest eine Glycosidrest aufweisende Organosiliciumverbindung aus Einheiten der Formel

$$R_aR^1{}_bSiO_{\frac{4-a-b}{2}} \qquad\qquad (I),$$

worin
R gleich oder verschieden sein kann und Wasserstoffatom oder organischer Rest bedeutet,
a 0, 1, 2 oder 3 ist,
b 0, 1, 2 oder 3 ist und
$R^1$ gleich oder verschieden sein kann und einen Rest der Formel

$$Z-(R^2O)_c-R^3- \qquad\qquad (II)$$

bedeutet, worin
Z einen Glycosidrest bedeutet, der aus 1 bis 10 Monosaccharideinheiten aufgebaut ist,
$R^2$ gleich oder verschieden sein kann und Alkylenrest bedeutet,
c 0 oder eine Zahl von 1 bis 20 ist und
$R^3$ Alkylenrest bedeutet,
mit der Maßgabe, daß die Summe aus a und b kleiner oder gleich 3 ist und Organosiliciumverbindung aus Einheiten der Formel (I) pro Molekül mindestens einen Rest $R^1$ enthält, und zumindest ein Metalloxid enthalten sind.

2. O/W-Sonnenschutz emulsion nach Anspruch 1, **dadurch gekennzeichnet, dass** bei den Glycosidrest aufweisenden Organosiliciumverbindungen Z einen Glycosidrest bedeutet, der aus 1 bis 2 Monosaccharideinheiten aufgebaut ist.

3. O/W-Sonnenschutz emulsion nach Anspruch 1 oder 2, **dadurch gekennzeichnet, dass** es sich bei den Glycosidrest aufweisenden Organosiliciumverbindungen um solche der Formel

$$R^1{}_xR_{3-x}SiO-[(SiRR^1O)_m- (SiR_2O)_n]_y-SiR_{3-x}R^1{}_x \qquad\qquad (III),$$

handelt, worin R und $R^1$ die in Anspruch 1 genannte Bedeutung haben,
m gleich oder verschieden sein kann und 0 oder eine Zahl von 1 bis 200 ist,
n gleich oder verschieden sein kann und 0 oder eine Zahl von 1 bis 1000 ist,
x 0 oder 1 ist und
y 0 oder eine Zahl von 1 bis 1200 ist,
mit der Maßgabe, daß die Verbindung der Formel (III) mindestens einen Rest $R^1$ aufweist.

4. O/W-Sonnenschutz emulsion nach Anspruch 3, **dadurch gekennzeichnet, dass** bei den Glycosidrest aufweisenden Organosiliciumverbindungen, falls in Formel (III) m durchschnittlich verschieden 0 ist, x 0 bedeutet.

5. O/W-Sonnenschutz emulsion nach Anspruch 3, **dadurch gekennzeichnet, dass** bei den Glycosidrest aufweisenden Organosiliciumverbindungen, falls in Formel (III) x durchschnittlich verschieden 0 ist, m 0 bedeutet.

6. O/W-Sonnenschutz emulsion nach einem oder mehreren der Ansprüche 1 bis 5, **dadurch gekennzeichnet, dass** es sich bei dem Metalloxid um eines oder mehrere aus der Gruppe handelt, die aus den Oxiden des Titans, Zinks, Eisens oder Aluminiums besteht.

7. Verfahren zur Herstellung der O/W-Sonnenschutz emulsion nach einem oder mehreren der Ansprüche 1-6, **dadurch gekennzeichnet, dass** eine O/W-Emulsion hergestellt wird, zu der bei einer Temperatur von unter 50 °C die in einem Lösungsmittel gelöste Glycosidrest aufweisende Organosiliciumverbindung mit dem Metalloxid hinzugefügt wird.

8. Kosmetische Zubereitung auf der Basis der O/W-Sonnenschutz emulsion nach einem oder mehreren der Ansprüche 1-6.

9. Kosmetische Zubereitung nach Anspruch 8, **dadurch gekennzeichnet, dass** es sich um Lotionen oder Cremes handelt.

## Claims

1. O/W sun protection emulsion wherein at least one organosilicon compound having a glycoside radical and composed of units of the formula

$$R_aR^1{}_bSiO_{\frac{4-a-b}{2}} \qquad\qquad (I),$$

in which
R may be identical or different and is a hydrogen atom or organic radical,
a is 0, 1, 2 or 3,
b is 0, 1, 2 or 3 and
$R^1$ may be identical or different and is a radical of the formula

$$Z\text{-}(R^2O)_c\text{-}R^3\text{-} \qquad (II)$$

in which
Z is a glycoside radical which is made up of 1 to 10 monosaccharide units,
$R^2$ may be identical or different and is an alkylene radical,
c is 0 or a number from 1 to 20 and
$R^3$ is an alkylene radical,
with the proviso that the sum of a and b is less than or equal to 3 and the organosilicon compound of units of the formula (I) contains at least one radical $R^1$ per molecule, and at least one metal oxide are present.

2. The O/W sun protection emulsion according to Claim 1, wherein, for the organosilicon compounds having a glycoside radical, Z is a glycoside radical which is made up of 1 to 2 monosaccharide units.

3. The O/W sun protection emulsion according to Claim 1 or 2, wherein the organosilicon compounds having a glycoside radical are those of the formula

$$R^1{}_xR_{3-x}SiO\text{-}[(SiRR^1O)_m\text{-}(SiR_2O)_n]_y\text{-}SiR_{3-x}R^1{}_x \qquad (III),$$

in which R and $R^1$ have the meanings given in Claim 1,
m may be identical or different and is 0 or a number from 1 to 200,
n may be identical or different and is 0 or a number from 1 to 1000,
x is 0 or 1 and
y is 0 or a number from 1 to 1200,
with the proviso that the compound of the formula (III) has at least one radical $R^1$.

4. The O/W sun protection emulsion according to Claim 3, wherein for the organosilicon compounds having a glycoside radical, if, in formula (III), m is on average different from 0, x is 0.

5. The O/W sun protection emulsion according to Claim 3, wherein for the organosilicon compounds having a glycoside radical, if, in formula (III), x is on average different from 0, m is 0.

6. The O/W sun protection emulsion according to one or more of Claims 1 to 5, wherein the metal oxide is one or more from the group which consists of the oxides of titanium, zinc, iron or aluminium.

7. Method for preparing the O/W sun protection emulsion according to one or more of Claims 1-6, **characterized in that** an O/W emulsion is prepared, to which, at a temperature of less than 50°C, the organosilicon compound having a glycoside radical dissolved in a solvent is added with the metal oxide.

8. Cosmetic preparation based on the O/W sun protection emulsion according to one or more of Claims 1-6.

9. Cosmetic preparation according to Claim 8, **characterized in that** it is in the form of a lotion or cream.


**Revendications**

1. Emulsion antisolaire H/E, **caractérisée en ce qu'**elle contient au moins un composé organosilicié comportant un reste glycoside, à base de motifs de formule

$$R_a R^1_b SiO_{\frac{4-a-b}{2}} \qquad\qquad (I)$$

dans laquelle
R peut être le même ou différent et représente un atome d'hydrogène ou un radical organique,
a est 0, 1, 2 ou 3,
b est 0, 1, 2 ou 3 et
$R^1$ peut être le même ou différent et représente un radical de formule

$$Z\text{-} (R^2O)_c\text{-}R^3\text{-} \qquad (II)$$

dans laquelle
Z représente un reste glycoside qui est constitué de 1 à 10 unités monosaccharidiques,
$R^2$ peut être le même ou différent et représente un radical alkylène,
c est 0 ou un nombre allant de 1 à 20 et
$R^3$ représente un radical alkylène,
étant entendu que la somme de a et b est inférieure ou égale à 3, et que le composé un composé organosilicié à base de motifs de formule (I) contient au moins un radical $R^1$, et au moins un oxyde métallique.

2. Emulsion antisolaire H/E selon la revendication 1, **caractérisée en ce** dans les composés organosiliciés comportant le reste glycoside, Z représente un reste glycoside qui est constitué de 1 ou 2 unités monosaccharidiques.

3. Emulsion antisolaire H/E selon la revendication 1 ou 2, **caractérisée en ce que** les composés organosiliciés comportant le reste glycoside consistent en ceux de formule

$$R^1R_{3-x}SiO\text{-} [(SiRR^1O)_m\text{-} (SiR_2O)_n]_y\text{-}SiR_{3-x}R^1_x \qquad (III)$$

dans laquelle R et $R^1$ ont les significations données dans la revendication 1,
m peut être le même ou différent et est 0 ou un nombre de 1 à 200,
n peut être le même ou différent et est 0 ou un nombre de 1 à 1 000,
x est 0 ou 1,
y est 0 ou un nombre de 1 à 1200,
étant entendu que le composé de formule (III) comporte au moins un radical $R^1$.

**4.** Emulsion antisolaire H/E selon la revendication 3, **caractérisée en ce** dans les composés organosiliciés comportant le reste glycoside, si dans la formule (III) m est en moyenne différent de 0, x représente 0.

**5.** Emulsion antisolaire H/F selon la revendication 3, **caractérisée en ce** dans les composés organosiliciés comportant le reste glycoside, si dans la formule (III) x est en moyenne différent de 0, m représente 0.

**6.** Emulsion antisolaire H/E selon une ou plusieurs des revendications 1 à 5, **caractérisée en ce** l'oxyde métallique consiste en un ou plusieurs oxydes choisis dans le groupe constitué par les oxydes du titane, du zinc, du fer et de l'aluminium.

**7.** Procédé pour la préparation de l'émulsion antisolaire H/E selon une ou plusieurs des revendications 1 à 6, **caractérisé en ce qu'**on prépare une émulsion H/E à laquelle, à une température inférieure à 50°C, on ajoute avec l'oxyde métallique le composé organosilicié comportant un reste glycoside, dissous dans un solvant.

**8.** Préparation cosmétique à base de l'émulsion antisolaire H/E selon une ou plusieurs des revendications 1 à 6.

**9.** Préparation cosmétique selon la revendication 8, **caractérisée en ce qu'**il s'agit de lotions ou de crèmes.